# FASCICULE DE BREVET EUROPEEN

(11) **EP 2 852 393 B1**
(45) Date de publication et mention de la délivrance du brevet: **13.09.2017**
(21) Numéro de dépôt: 13714953.0
(22) Date de dépôt: 10.04.2013
(51) Int. Cl.: A61K 35/741, A61K 35/644, A61K 33/06, A61P 43/00, A23L 21/20, A23L 33/135

(54) **COMPOSITIONS À BASE DE PROBIOTIQUES ET D'UN COMPLEXE BEEPOLLEN/ARGILE, LEUR PRÉPARATION ET LEURS UTILISATIONS EN NUTRITION ET THÉRAPEUTIQUE**
ZUSAMENSETZUNG BEINHALTEND PROBIOTIKA UND EINEN KOMPLEX AUS BEEPOLLEN UND TON, VORBEREITUNG SOWIE ANWENDUNGEN IN ERNÄHRUNGSTHERAPIE UND THERAPIE
COMPOSITIONS COMPRISING PROBIOTICS AND A COMPLEX BEEPOLLEN/CLAY, PREPARATION AND USES IN NUTRITION AND THERAPY

(30) Priorité: 10.04.2012 FR 1253253
(43) Date de publication de la demande: 01.04.2015
(73) Titulaire: Laboratoire Beepratte, 75006 Paris (FR); Fregonese, Alexandra, 47310 Moncaut (FR)
(72) Inventeur: FREGONESE, Alexandra, F-47310 Moncaut (FR)
(74) Mandataire: Lavoix
(86) Numéro de dépôt international: PCT/EP2013/057496
(87) Numéro de publication internationale: WO 2013/153117

(56) Documents cités:
- EP-A1- 0 191 128
- WO-A1-2012/080333
- US-A1- 2008 031 940
- YU N ET AL: "Method for preparing tumor mutation-resisting yoghourt with micron ash tree pollen, pine pollen, bee pollen and ganoderma lucidum spore powder", WPI / THOMSON,, vol. 2006, no. 76, 26 juillet 2006 (2006-07-26), XP002634574,
- DATABASE WPI Week 200939 Thomson Scientific, London, GB; AN 2009-J49211 XP002689030, "Functional yogurt including stirring, jelly-type, or drink-type yogurt, contains liquid cream, bee pollen and probiotic", & CN 101 422 196 A (SHANGHAI SHUANGJIN BIOTECHNOLOGY CO LTD) 6 mai 2009 (2009-05-06)
- DATABASE WPI Week 200215 Thomson Scientific, London, GB; AN 2002-111739 XP002689031, "Bentonite (or montmorillonite) containing various kinds of colloidal trace minerals for foods and medical supplies", & KR 2001 0079473 A (KIM G H) 22 août 2001 (2001-08-22)
- DATABASE WPI Week 200837 Thomson Scientific, London, GB; AN 2008-F73322 XP002689032, & KR 100 707 342 B1 (KOREA PEDIATRIC CANCER FOUND) 13 avril 2007 (2007-04-13)
- DATABASE BIOSIS [Online] BIOSCIENCES INFORMATION SERVICE, PHILADELPHIA, PA, US; 2009, VASQUEZ ALEJANDRA ET AL: "The lactic acid bacteria involved in the production of bee pollen and bee bread", XP002689033, Database accession no. PREV200900447603 & JOURNAL OF APICULTURAL RESEARCH, vol. 48, no. 3, 2009, pages 189-195, ISSN: 0021-8839, DOI: 10.3896/IBRA.1.48.3.07

## Description

La présente invention concerne l'administration de microorganismes (bactéries ou levures) à visée probiotique au moyen d'un support à base de beepollen et d'argile, ainsi que les compositions comprenant lesdits microorganismes et ledit complexe beepollen/argile.

Selon la présente invention, le terme microorganisme désigne des microorganismes (bactéries ou levures) à visée probiotique.

Les probiotiques sont des microorganismes tels que les bactéries ou les levures, non pathogènes et non toxiques qui, délivrés vivants en quantité suffisante dans l'intestin, contribuent au maintien de l'équilibre de la flore et au renforcement de la muqueuse intestinale. Ils sont actuellement très recherchés pour les effets bénéfiques qu'ils apporteraient en terme de nutrition et de santé.

Ainsi, la consultation d'experts d'octobre 2001 de Food and Agricultural Organization (FAO)/OMS sur l'évaluation de la santé et des propriétés nutritionnelles des probiotiques a défini les probiotiques comme des « *microorganismes vivants qui, lorsqu'ils sont administrés en quantité suffisante, confèrent un bénéfice pour la santé de celui qui les consomme* » et a rapporté que les probiotiques peuvent jouer un rôle important dans les fonctions immunologiques, digestives et respiratoires et pourraient avoir un effet significatif pour traiter les maladies infectieuses, notamment chez les enfants.

Les microorganismes à visée probiotique commercialement disponibles sont généralement lyophilisés et conditionnés sous différentes formes (gélules, sachets, etc.). Néanmoins, l'industrialisation, l'acidité gastrique et la mise en contact avec les sucs biliaires et pancréatiques peuvent être responsables de la perte d'une grande majorité de ces microorganismes, lesquels doivent par ailleurs être réactivés s'ils ont été lyophilisés. Dans la littérature, plusieurs études ont été consacrées à la viabilité des probiotiques en milieu acide. Ainsi, les effets délétères de l'acidité sur la survie de certaines souches probiotiques (*Bifidobacterium* et *Lactobacillus*) ont été rapportés par Sun et al. International Journal of Food Microbiology, 61,17-25 (2000), Sultana et al. International Journal of Food Microbiology, 62, 47-55 (2000), Favaro-Trindade et al. Journal of Microencapsulation, 19(4), 485-494 (2002) et Hansen et al. Food Microbiology, 19, 35-45 (2002) notamment.

Même parmi les lactobacilles utilisés par l'industrie laitière pour la fabrication de lait fermenté et de yaourts, un grand nombre ne survit pas à l'environnement acide de l'estomac et ne peut donc prétendre à la définition des probiotiques. En effet, pour être qualifié de « probiotique », une souche de microorganismes doit arriver vivante en quantité suffisante au niveau intestinal et s'établir dans la microflore intestinale.

Compte tenu de l'intérêt des probiotiques, il est donc désirable de mettre à disposition des formulations permettant aux microorganismes d'arriver vivants et en quantité suffisante dans l'intestin de l'hôte.

Ainsi, il est essentiel de fournir des compositions qui garantissent l'effet probiotique des microorganismes ajoutés au beepollen, en assurant leur biodisponibilité et leur activité biologique.

La demande internationale PCT/EP2011/072756 décrit des combinaisons de beepollen et d'argile, nommées ici complexe beepollen/argile, permettant la stabilisation, la formulation sous forme de doses et l'administration de beepollen frais, mais n'envisage aucunement son utilisation à titre de support pour l'administration des microorganismes à visée probiotique caractérisés et préalablement mis sous culture, notamment lyophilisés.

Il a maintenant été découvert, et c'est l'un des objets de la présente invention, que lesdites combinaisons d'argile et de beepollen constituent un système d'administration idéal des microorganismes à visée probiotique, en leur donnant la protection nécessaire contre l'acidité stomacale, les sucs biliaires et pancréatiques ; en fournissant l'alimentation indispensable à leur développement et, préalablement à leur réactivation dans le cas de microorganismes lyophilisés, en leur permettant de donner naissance intra corpus à des microorganismes d'une grande vitalité, naturellement adaptés à l'environnement digestif ; en les délivrant vivants à l'intestin , en les y fixant et en les y libérant.

Selon un premier objet, la présente invention concerne donc une composition comprenant :
- un ou plusieurs microorganismes à visée probiotique caractérisés et mis sous culture avant leur formulation et qui, lorsqu'ils sont délivrés vivants en quantité suffisante à l'intestin, confèreraient un bénéfice pour la santé et/ou la nutrition de l'hôte, et
- un complexe à base de beepollen et d'argile.

Selon un autre objet, la présente invention concerne également l'utilisation d'un complexe à base de beepollen/argile à titre de support pour l'administration de microorganismes à visée probiotique caractérisés et mis sous culture avant leur formulation et qui, lorsqu'ils sont délivrés vivants en quantité suffisante à l'intestin, confèreraient un bénéfice pour la santé et/ou la nutrition de l'hôte notamment lyophilisés, en particulier pour améliorer la biocharge et la biodisponibilité desdits microorganismes.

Ainsi, selon l'invention, il a été démontré que le complexe beepollen/argile constitue un milieu de protection et de nutrition idéal pour les microorganismes à visée probiotique capable d'assurer leur protection lors du passage gastrique, de la mise en contact avec les sucs biliaires et pancréatiques de fournir l'alimentation nécessaire à leur développement et, préalablement à leur réactivation dans le cas de microorganismes lyophilisés.

Le complexe permet de maintenir les microorganismes vivants jusqu'à l'intestin et de conserver leurs caractéristiques biologiques, et par conséquent leurs bienfaits physiologiques sur l'équilibre de la flore intestinale de l'hôte.

En particulier, le complexe beepollen/argile offre un environnement favorable à la naissance intra corpus de microorganismes vivants à visée probiotique d'une grande vitalité, adaptés à l'environnement digestif. De plus, alors que la survie des microorganismes à visée probiotique devient problématique en dessous d'un pH de 3, il a été démontré que le support beepollen/argile des compositions selon l'invention permet de protéger les microorganismes de l'acidité gastrique, en maintenant le pH à un niveau compatible à la vie cellulaire par effet tampon qui isole ainsi les microorganismes du pH stomacal et qui permet la naissance intra corpus de microorganismes à l'issue du passage gastrique. Il a également été démontré que cette protection se prolonge lors du passage dans l'intestin.

On entend par naissance intra corpus la capacité des microorganismes à se multiplier dans le complexe beepollen/argile et à s'adapter à l'environnement physiologique (notamment les conditions du système digestif) de l'hôte à qui ils sont administrés.

On entend par « complexe beepollen/argile » toute matière stable ou « anhydre » résultant de la fixation de beepollen provenant de quelque espèce végétale que ce soit sur une structure lamellaire silicatée. La demande internationale PCT/EP2011/072756 décrit des combinaisons de beepollen et d'argile. Au sens de l'invention, on entend par « beepollen » le pollen prélevé par les abeilles. Il s'agit de l'élément fécondant végétal tiré par l'abeille de l'organe mâle de la fleur, aggloméré de fleur en fleur et transporté jusqu'à la ruche. Il peut être prélevé par tout moyen, et notamment juste avant son entrée dans la ruche (grâce à une trappe à pollen), selon les méthodes habituelles.

Au sens de l'invention, ce terme « beepollen » s'applique au pollen récolté par les abeilles quelque soit son mode de prélèvement et/ou conservation.

Au sens de l'invention, le terme « beepollen » désigne le beepollen sous toute forme permettant le maintien des caractéristiques du pollen frais. Il comprend donc notamment le beepollen frais ainsi que le beepollen préalablement congelé.

Dans le cadre de la présente invention, le beepollen peut être originaire de diverses espèces végétales et n'est pas limité à une fleur particulière. On peut ainsi citer le beepollen de seigle, de maïs, de tournesol, de ciste, de châtaigner, de kiwi, de saule, de coquelicot, de lavande, de bruyère, etc.

Le beepollen, notamment le beepollen congelé, peut être disponible commercialement par exemple chez Pollenergie.

Le beepollen peut également être de composition variable, notamment en fonction de la nature des plantes d'origine ainsi que de la période de la récolte, du mode de récolte ou encore de la zone géographique (pays, régions...).

Ainsi, le beepollen frais convenant à l'invention contient généralement de 5% à 36% d'eau et de 64% à 95% de matières sèches, parmi celles-ci :
- 2,5% à 3,8% de sels minéraux ;
- 4,2% à 19,8% de lipides ;
- 8% à 30% d'albumine ;
- 5% à 7% d'amidon ;
- des vitamines ;
- des facteurs de croissance ;
- de l'acide folique ;
- des polyphénols ;
- des phytostérols;
- des ferments et levures.

On entend par « argile » un matériau comprenant des minéraux argileux tels que les silicates hydratés, notamment des silicates d'aluminium ou de magnésium. On préfère notamment les phyllosilicates présentant une structure feuilletée constituée d'un ou plusieurs feuillets.

Selon la classification de Jozja, basée sur l'épaisseur et la structure du feuillet, on distingue quatre groupes d'argiles :
i) Minéraux à 7 Å : Son épaisseur est d'environ 7 Å, c'est la catégorie des kaolinites (kaolin, halloysite ...)
ii) Minéraux à 10 Å : Son épaisseur est d'environ 10 Å, c'est la catégorie des illites (illite, glauconite, ..)
iii) Minéraux à 14 Å : Son épaisseur est d'environ 14 A, c'est la catégorie des smectites (montmorillonite, bentonite, ghassoul ...)
iv) Minéraux fibreux : L'épaisseur du feuillet est variable on les appelle chlorites ou minéraux fibreux (sepiolite, attapulgite ...).

Le terme « argile » selon l'invention fait donc référence à ces quatre types d'argile. A titre de minéraux argileux, on peut notamment citer l'illite, la chlorite, la glauconite, la kaolinite, l'attapulgite, la sépiolite et la montmorillonite. On préfère notamment, à titre d'argile, le kaolin, l'illite, la montmorillonite, l'attapulgite, et plus particulièrement la montmorillonite.

Au sens de l'invention, on entend par microorganisme, un organisme microscopique incluant notamment les bactéries et les levures, notamment non pathogènes et non toxiques.

Au sens de l'invention, on entend par microorganisme à visée probiotique, des microorganismes caractérisés et mis sous culture avant leur formulation, qui, lorsqu'ils sont délivrés vivants en quantité suffisante à l'intestin, confèreraient un bénéfice pour la santé et/ou la nutrition de l'hôte.

Le terme « caractérisé » utilisé ici fait référence à un microorganisme dont la ou les souches qui le constituent ont été identifiée(s), notamment de façon à connaître la nature de la ou les souche(s) et éventuellement leur(s) concentration(s).

Selon un mode de réalisation, les microorganismes caractérisés selon l'invention sont ajoutés au beepollen. Selon un mode de réalisation, les microorganismes ajoutés au beepollen sont distincts, notamment de par leur nature, nombre, structure, des microorganismes naturellement présents dans le beepollen. Selon un mode de réalisation, des microorganismes lyophilisés sont ajoutés.

A titre de levure, on peut citer *Saccharomyces cerivisiae.* A titre de bactéries, on peut citer essentiellement les bactéries lactiques, telles que les lactobacilles, les bifidobactéries ou autres bactéries lactiques.

Selon l'invention :
- à titre de lactobacilles, on peut notamment citer *Lactobacillus acidophilus, Lactobacillus amylovirus, Lactobacillus brevis, Lactobacillus casei, Lactobacillus cellobius, Lactobacillus crispatus, Lactobacillus curvatus, Lactobacillus delbrueckii, Lactobacillus farciminis, Lactobacillus fermentum, Lactobacillus gasseri, Lactobacillus gallinarum, Lactobacillus helveticus, Lactobacillus johnsonii, Lactobacillus paracasei, Lactobacillus plantarum, Lactobacillus reuteri, Lactobacillus rhamnosus, Lactobacillus lactis.*
- à titre de bifidobactéries, on peut citer *Bifidobacterium adolescentis, Bifidobacterium bifidum, Bifidobacterium breve, Bifidobacterium infantis, Bifidobacterium lactis* (reclassé *Bifidobacterium animalis*)*, Bifidobacterium laterosporus, Bifidobacterium longum, Bifidobacterium thermophilum.*
- d'autres bactéries lactiques incluent notamment *Enterococcus faecalis, Enterococcus faecium, Lactococcus lactis, Leuconostoc mesenteroides, Pediococcus acidilactici, Sporolactobacillus inulinus, Streptococcus diacetylactis, Streptococcus intermedius, Streptococcus thermophilus.*
- à titre de bactéries non lactiques, on peut citer *Bacillus spp, Escherichia coli (souche nissle), Propionibacterium freudenreichii.*

On préfère notamment les lactobacilles et bifidobactéries telles que les espèces *Bifidobacterium bifidum* (bifidus), *Bifidobacterium lactis, Bifidobacterium longum, Bifidobacterium breve, Lactobacillus reuteri, Lactobacillus acidophilus, Lactobacillus casei, Lactobacillus plantarum, Lactobacillus rhamnosus.*

Ces microorganismes à visée probiotique sont généralement disponibles commercialement.

Lesdits microorganismes convenant à l'invention sont généralement isolés, lyophilisés, caractérisés et/ou mis sous culture avant d'être ajoutés et inclus dans le complexe beepollen/argile.

De fait, selon un mode de réalisation préféré cette définition ne couvre pas les microorganismes naturellement présents dans le complexe beepollen/argile, et qui n'auraient pas été isolés, caractérisés, mis sous culture et/ou lyophilisés préalablement à leur inclusion dans le complexe.

Les microorganismes lyophilisés à visée probiotique rentrent dans le champ de la présente invention.

Les microorganismes à visée probiotique sont majoritairement commercialisés sous forme lyophilisée. La lyophilisation, ou séchage à froid, est un procédé basé sur la sublimation qui permet de retirer l'eau contenue dans une matière, un aliment ou un produit afin de le rendre stable à la température ambiante et ainsi faciliter sa conservation.

La lyophilisation comporte de nombreux avantages par rapport aux autres procédés de séchage ou de conservation.
- La lyophilisation permet de conserver une grande partie des qualités des aliments étant donné que ceux-ci demeurent à une température sous le point de congélation durant la sublimation. L'utilisation de la lyophilisation est particulièrement importante dans le cas des bactéries lactiques puisqu'elles sont très sensibles à la chaleur.
- Les aliments lyophilisés en général ne nécessitent pas de réfrigération pour se conserver. Les coûts d'entreposage et de transport peuvent être ainsi réduits de façon appréciable.
- La lyophilisation entraîne une diminution importante du poids, ce qui facilite grandement le transport des aliments lyophilisés. Par exemple, plusieurs aliments contiennent jusqu'à 90% d'eau. Ils seront donc dix fois plus légers après lyophilisation.
- La plupart des aliments lyophilisés se réhydratent très rapidement grâce à leur texture poreuse. En effet, la lyophilisation n'entraîne pas de diminution de volume appréciable. L'eau peut donc reprendre sa place facilement dans la structure moléculaire de l'aliment.

Les microorganismes lyophilisés sont mis en état dormant par dessication. Ils doivent donc être réactivés pour arriver vivants dans l'intestin et y exercer une action bénéfique pour la santé de l'hôte, conformément à l'invention.

Selon l'invention, il a été démontré que le complexe beepollen/argile représente un support permettant la réactivation de ces bactéries lyophilisées.

Ce support représente donc un environnement favorable et idéal à la nutrition, la protection, au développement et pour les microorganismes lyophilisés la réactivation des microorganismes, y compris lors de l'administration par voie orale.

Généralement, les compositions selon la revendication comprennent :
- de 0,1% à 70% de probiotiques lyophilisés ; et
- de 30% à 99,9% dudit complexe,
les pourcentages s'entendant en poids ;
et plus particulièrement :
- de 3% à 30% de probiotiques lyophilisés ; et
- de 70% à 97% dudit complexe,
les pourcentages s'entendant en poids.

Les complexes beepollen/argile comprennent généralement :
- de 4% à 90% de beepollen, et
- de 10% à 96% d'argile.

Les pourcentages s'entendant en poids.

Plus préférentiellement, les complexes selon l'invention comprennent :
- de 20% à 40% de beepollen et
- de 60% à 80% d'argile,
et plus particulièrement :
- de 30% à 35% de beepollen et
- de 65% à 70% d'argile.

Les complexes selon l'invention présentent avantageusement une granulométrie inférieure à 500 µm, un taux d'humidité inférieur à 15% et/ou une activité d'eau (aw) inférieure à 0,75.

Généralement, les compositions selon l'invention se présentent sous forme de poudre et présentent notamment les caractéristiques suivantes :

| **ANALYSES** | **SPECIFICATIONS** |
|---|---|
| ASPECT | Poudre |
| COULEUR | Gris-vert foncé à gris-vert clair selon la quantité de bactéries lyophilisées |
| ODEUR | Spécifique |
| PH | NA |
| DENSITE | NA |
| METAUX LOURDS (Pb, As) | - Pb < 2ppm |
| | - As < 1ppm |

Avantageusement, les compositions selon l'invention peuvent être formulées sous forme gastro-résistante, notamment sous forme de gélules gastro-résistantes.

Selon l'invention, il a également été démontré, de façon inattendue, qu'une formulation gastro-résistante de microorganismes à visée probiotique et du complexe beepollen/argile a permis l'augmentation de la biocharge avec la naissance intra corpus de ferments alors que les microorganismes formulés sous forme de gélules gastro-résistantes, sans le complexe beepollen/argile, ont un niveau de viabilité considérablement affecté.

Avantageusement, lorsque le complexe beepollen/argile chargé en probiotiques lyophilisés est formulé en gélules gastro-résistantes, le passage au niveau de l'estomac s'inscrit comme un facteur favorable à la prolifération. Au contact du flux gastrique contenu dans l'estomac, la gélule gastro-résistante devient perméable, l'eau pénètre et apporte l'humidité dont les microorganismes ont besoin pour entamer la phase de prolifération.

Selon l'invention, l'utilisation d'un complexe beepollen/argile comme support pour l'administration orale de microorganismes à visée probiotique permet de
- protéger les microorganismes à visée probiotique des effets délétères du conditionnement ;
- réactiver les microorganismes à visée probiotique lyophilisés inclus dans la composition ;
- nourrir et maintenir viables les microorganismes à visée probiotique inclus dans la composition ;
- protéger les microorganismes à visée probiotique de l'acidité gastrique
- protéger les microorganismes à visée probiotique des sucs biliaires et pancréatiques ;
- donner naissance intra corpus à des microorganismes à visée probiotique adaptés à l'environnement digestif ;
- libérer les microorganismes dans le milieu intestinal tout en favorisant leur adhésion et interaction avec la muqueuse intestinale.

Les microorganismes doivent se fixer à l'intestin pour contribuer au renforcement de la barrière intestinale et exercer leur action bénéfique sur la santé de l'hôte. Ainsi, par exemple, les bifidobactéries possèdent des capacités d'adhésion à la muqueuse intestinale où elles reconnaissent différents récepteurs de l'épithélium intestinal. Les bactéries lactiques, quant à elles, occupent des sites potentiels de colonisation par les pathogènes en empêchant la pénétration d'antigènes nuisibles et en évitant ainsi la prolifération de germes pathogènes. Elles consomment les nutriments disponibles limitant la source de nutriments pour les pathogènes et entrent en compétition avec les pathogènes pour l'accès aux récepteurs de l'hôte. De plus, elles sécrètent des molécules antimicrobiennes telles les bactériocines au niveau de la surface épithéliale et agissent aussi sur le milieu (p. ex. acidification).

En augmentant la rétention des microorganismes à visée probiotique dans l'intestin, le complexe beepollen/argile favorise l'interaction bénéfique de ces microorganismes avec la muqueuse intestinale. Le microbiote intestinal exerce de nombreuses fonctions physiologiques nécessaires et bénéfiques pour le maintien de la santé de l'hôte. Le maintien de l'équilibre de la flore intestinale et le renforcement de la barrière intestinale présentent un intérêt majeur. Les microorganismes à visée probiotique sont de bons candidats pour atteindre cet objectif. Cependant, des difficultés majeures empêchent généralement les microorganismes d'exercer une activité probiotique : la nécessité d'alimenter correctement les microorganismes pour les maintenir en vie ; la nécessité de réactiver les microorganismes lyophilisés ; la faible résistance des microorganismes aux chocs de l'industrialisation ; la faible résistance des microorganismes à l'acidité gastrique, aux sucs biliaires et pancréatiques ; le manque d'adaptation des microorganismes à l'environnement digestif et le caractère planctonique des microorganismes qui est un frein à leur adhésion à la muqueuse et à leur interaction avec elle. Des stratégies de contournement de ces difficultés existent, mais aucune ne permet de contourner l'ensemble des difficultés. Par exemple, des souches bactériennes bénéfiques isolées et mises sous culture pour leur résistance à l'acidité gastrique ne sont pas réactivées, et le pourcentage infime qui va atteindre l'intestin ne pourra ni s'y fixer ni interagir efficacement au niveau intestinal. Aujourd'hui, aucun support ne permet de résoudre en simultané les différents freins biologiques et/ou physiologiques en garantissant une action sur le site cible. La présente invention permet de s'affranchir dans une large mesure de ces difficultés et fournit aux microorganismes l'environnement nécessaire à leur action probiotique au niveau intestinal.

Le complexe beepollen/argile permet de s'affranchir simultanément de l'ensemble des obstacles industriels, biologiques et/ou physiologiques à l'action des probiotiques en apportant les éléments suivants :
- Environnement nutritif nécessaire à la subsistance des bactéries
- Environnement nutritif nécessaire à la réactivation des bactéries lyophilisées
- Résistance à l'acidité gastrique
- Résistance aux sucs biliaires et pancréatiques
- Adaptation à l'environnement digestif
- Fixation à la paroi intestinale et délivrance.

L'invention concerne également un procédé de préparation d'une composition selon l'invention. Ledit procédé comprend :
- le mélange de beepollen et d'argile ; et
- l'ajout et le mélange de microorganismes à visée probiotique.

Lesdits microorganismes à visée probiotique peuvent être lyophilisés, présentés sous forme de poudre notamment.

De préférence, le mélange obtenu peut être tamisé pour obtenir la granulométrie désirée. Avantageusement, un tamis de maillage 500µm est utilisé.

L'invention concerne également l'utilisation d'un complexe à base de beepollen et d'argile pour véhiculer les microorganismes à visée probiotique caractérisés et mis sous culture avant leur formulation et qui, lorsqu'ils sont délivrés vivants en quantité suffisante à l'intestin, confèreraient un bénéfice pour la santé et/ou la nutrition de l'hôte jusqu'à l'intestin, les fixer, améliorer leur biodisponibilité et/ou leur activité biologique probiotique au niveau intestinal. Ledit complexe constitue donc un biovecteur pour l'administration de microorganismes à visée probiotique.

On entend ici par « activité biologique » l'effet nutritionnel (notamment diététique) et/ou physiologique, tel que probiotique et thérapeutique, exercé par lesdits microorganismes chez les sujets qui les ont consommés.

La présente invention concerne donc notamment une composition alimentaire, cosmétique, pharmaceutique et/ou galénique et/ou un dispositif médical comprenant une composition selon l'invention.

Lesdites compositions/dispositifs selon l'invention sont donc utiles à titre préventif et/ou curatif, thérapeutique et/ou nutritionnel (notamment diététique), pour usage humain et/ou animal. Les excipients nécessaires à la mise en forme industrielle du produit peuvent être choisis parmi toutes les matières acceptables selon la réglementation (aliment, complément alimentaire, aliment fonctionnel, cosmétique, dispositif médical, pharmaceutique) qui régit ce produit.

On peut ainsi notamment citer comme excipients les dérivés de la cellulose ou de la cellulose microcristalline, les carbonates alcalino-terreux, le phosphate de magnésium, les amidons, les amidons modifiés, le lactose pour les formes solides, l'eau, les solutés aqueux, le sérum physiologique, les solutés isotoniques, etc.

Selon un mode de réalisation, les compositions comprennent également une source de sucre, tel que le glucose permettant aux bactéries de se développer.

Les compositions selon l'invention peuvent être présentées sous des formes destinées à l'administration par voie orale, sublinguale, topique, locale, intratrachéale, intranasale ou rectale, notamment orale.

Elles peuvent donc être notamment présentes sous forme de solutés ou flacons multi-doses, ou sous forme de comprimés nus ou enrobés, de dragées, de capsules, de gélules molles ou dures, de granules, pilules, de cachets, de poudres, de suppositoires ou de capsules rectales, de solutions ou de suspensions, ou encore de crèmes, gels, pommades, pâtes, patch, produits alimentaires tels que yaourts, etc.

La présente invention vise plus particulièrement les formulations gastrorésistantes telles que les pâtes ou les gélules, notamment les gélules gastrorésistantes comprenant une composition selon l'invention.

De préférence, ladite composition contient une quantité efficace de la composition selon l'invention.

La dose et/ou posologie peut varier dans les limites importantes (0,5 mg à 1000 mg) et peut dépendre de la voie d'administration, ainsi que de l'âge et du poids du sujet.

Il peut y avoir des cas particuliers où des dosages plus élevés ou plus faibles sont appropriés ; de tels dosages ne sortent pas du cadre de l'invention. Selon la pratique habituelle, le dosage approprié à chaque sujet est déterminé selon le mode d'administration, le poids et la réponse dudit sujet, ainsi que la concentration des microorganismes dans ladite composition.

Les compositions alimentaires selon l'invention peuvent être notamment un aliment, un aliment fonctionnel (ou alicament) ou un complément alimentaire.

Les aliments fonctionnels ou alicaments sont généralement définis comme un aliment conventionnel, ou qui en a l'apparence, qui fait partie de l'alimentation normale, et qui a pour caractéristique de procurer des effets physiologiques bénéfiques dépassant ses fonctions nutritionnelles habituelles ou de réduire le risque de maladies chroniques.

L'invention vise donc également les aliments fonctionnels tels que les yaourts comprenant une composition selon l'invention.

Ladite composition peut aussi constituer un complément alimentaire au sens de la Directive Européenne 2002/46/CE.

Ledit complément alimentaire est avantageusement formulé sous forme de dose, à savoir dans une forme de présentation définie par la Directive Européenne 2002/46/CE, telle que « *les gélules, les pastilles, les comprimés, les pilules et autres formes similaires, ainsi que les sachets de poudre, les ampoules de liquide, les flacons munis d'un compte-goutte et les autres formes analogues de préparations liquides ou en poudre destinées à être prises en unités de faible quantité* »*.*

Ladite composition peut aussi constituer notamment une spécialité pharmaceutique au sens de la Directive Européenne 2001/83/CE.

Ladite composition peut être utile dans la prévention et/ou le traitement de désordres dans lesquels les probiotiques sont généralement considérés bénéfiques. On peut ainsi citer notamment l'ostéoporose, des maladies cardio-vasculaires, des cancers, des désordres du foie et du métabolisme, la fatigue, des troubles circulatoires, des troubles articulaires, les symptômes ménopausiques, des désordres du système nerveux, des désordres du système gastro-intestinal, des désordres oculaires, urinaires, des désordres de la prostate tel que l'hypertrophie bénigne de la prostate, la prostatite chronique, la prostatodynie et le cancer de la prostate, les troubles nutritionnels, dermatologiques et cosmétodermatologiques, l'asthénie et la fatigabilité, la convalescence et la post-chirurgie, les déséquilibres hydro-électrolytiques, les améliorations des conditions physiques, etc.

Les compositions pharmaceutiques selon l'invention peuvent également comprendre un ou plusieurs ingrédients actifs.

La présente invention concerne également les dispositifs médicaux comprenant une composition selon l'invention. Ladite composition peut donc constituer un dispositif médical au sens de la Directive Européenne 93/42 CEE.

### FIGURES

La figure 1 représente le développement de bactéries lyophilisées (*Bifidobacterium longum*) dans un milieu idéal et au sein du complexe beepollen/argile.
La figure 2 représente les pourcentages de gain et de perte de ferments après passage dans l'estomac pour quatre compositions formulées sous forme de gélules gastro-résistantes :
   i) sur support beepollen/argile ;
   ii) produit Bion Transit,
   iii) produit Lactibiane réf. 2,5G,
   iv) Arkoprobiotics défense +.
La figure 3 représente la survie des bactéries lyophilisées en gélules gastro-résistantes après passage dans l'estomac.
La figure 4 représente la réactivation des bactéries lyophilisées associées au complexe beepollen/argile, en gélules gastro-résistantes, après passage dans l'estomac.
La Figure 5 illustre les gains et pertes des ferments après passage dans l'estomac et action de la bile et du pancréas, avec et sans complexe.
La Figure 6 illustre l'effet synergique du beepollen et de l'argile sur les gains de ferments dans l'estomac, chyme, muqueuse et liquide intestinal.

Les exemples suivants illustrent l'invention, sans toutefois la limiter. Les produits de départs utilisés sont des produits connus ou préparés selon des modes opératoires connus.

Les pourcentages sont exprimés en poids, sauf mention contraire.

### EXEMPLES

### - Matériel:

Balance électronique de précision- Mettler Toledo PG803
Etuve WTB binder
Agitateur- IKA Labotechnik Eurostar
pH mètre HANNA instruments
Bécher en verre 250mL - Bomex
Spatules courbées, forme cuillère, acier inox 18/8 - Labo-moderne

### - Fournisseurs de matières premières:

Pollen de Tournesol congelé sous gaz azote
Pollenergie
La Grabère - 47450 St Hilaire de Lusignan
Tél. 05 53 68 11 11 - Fax. 05 53 68 11 12
Argile Montmorillonite
Argiletz
14 route d'Echampeau - 77 440 Lizy sur Ourcq
Tél. 01 60 61 20 88 - Fax. 01 60 61 27 39.

Le complexe a été préparé de la façon suivante.

Pour la préparation de 150 g de complexe beepollen/argile : les proportions de chacun des composants est de 66,66 % d'argile pour 33,33% de beepollen.
- Peser 100 g d'argile dans un bécher de 250 mL ;
- Peser 50 g de beepollen congelé dans un bécher de 250 mL ;
- Les pièces de l'agitateur sont de préférence sèches pour éviter l'hydratation du complexe ;
- Mélanger l'argile ;
- Ajouter le beepollen et laisser mélanger pendant 30 secondes.

La granulométrie désirée est obtenue par tamisage au moyen d'un tamis de maillage 500 µm.

Les compositions à base de beepollen/argile et de microorganismes à visée probiotique sont préparées de la façon suivante pour 166.67g de produit final avec 10% de microorganismes :
- Peser 100g d'argile dans un bécher de 250 mL;
- Peser 50g de beepollen dans un bécher de 250 mL;
- Peser 16.67g de microorganismes dans un bécher de 100 mL;
- Les pièces de l'agitateur sont de préférences sèches pour éviter l'hydratation du complexe;
- Mélanger l'argile;
- Ajouter le beepollen et laisser mélanger pendant 30 secondes;
- Ajouter les microorganismes et laisser mélanger pendant 30 secondes;

La granulométrie désirée est obtenue par un tamisage au moyen d'un tamis de maillage 500 µm.

### Exemple 1 : Mise en évidence de la capacité du complexe beepollen/argile à fournir un milieu de nutrition et réactivation des bactéries lyophilisées

Il a été rapporté, dans la littérature, les besoins en ingrédients indispensables à la réactivation des bactéries lyophilisées.

Ces ingrédients (sucres, protéines, sodium, etc.) et les proportions optimales (besoins en %) pour la réactivation des bactéries lyophilisées sont représentés dans les deux premières colonnes du tableau ci-dessous. La troisième colonne de ce tableau indique le pourcentage de ces différents ingrédients contenu dans le complexe beepollen/argile.

| **Ingrédients** | **Besoins en %** | **Dans le support beepollen/argile %** |
|---|---|---|
| Peptone (hydrolyse de protéine) | 16 | 35 |
| Protéine | 13 | |
| Levure La plus grande source de vitamine B1 (12mg/100g) nécessaire au métabolisme des glucides et des lipides | 6.6 | Entre 6 et 13 |
| Glucose | 32 | De 20 à 40% |
| Acétate de sodium trihydraté | 8 | Sodium apporté par l'argile |
| Citrate d'ammonium | 3.2 | Apporté par l'argile |
| Tween (solubilisant nécessaire à l'assemblage des substances non miscibles entre elles) | 1.6 | non applicable |
| Hydrogénophosphate de potassium | 3.2 | De 20 à 45% des cendres |
| Sulfate de magnésium heptahydraté | 0.3 | De 1 à 12% des cendres |
| Sulfate de manganèse tétrahydraté | 0.1 | 1.4% des cendres* |
| Agar (polysacharides) | 16 | Entre 13 et 55% |
| pH | 6.2 | Pollen pH6.0 maintenu acide par l'argile |
| | | De nombreuses vitamines, des acides aminés et des substances de croissance |

On retrouve donc une parfaite concordance entre les éléments indispensables à la réactivation des bactéries lyophilisées rapportés dans la littérature et les éléments contenus dans le support de beepollen/argile.

Le développement de bactéries *Bifidobacterium longum* au sein d'un milieu nutritif idéal et au sein du complexe beepollen/argile a été mis en évidence de la façon suivante.
1- Les bactéries ont été ensemencées dans un milieu de culture idéal et d'autres ont été ensemencées avec le complexe beepollen/argile en quantité équivalente.
2- Des dénombrements ont été réalisés pour ces deux échantillons.

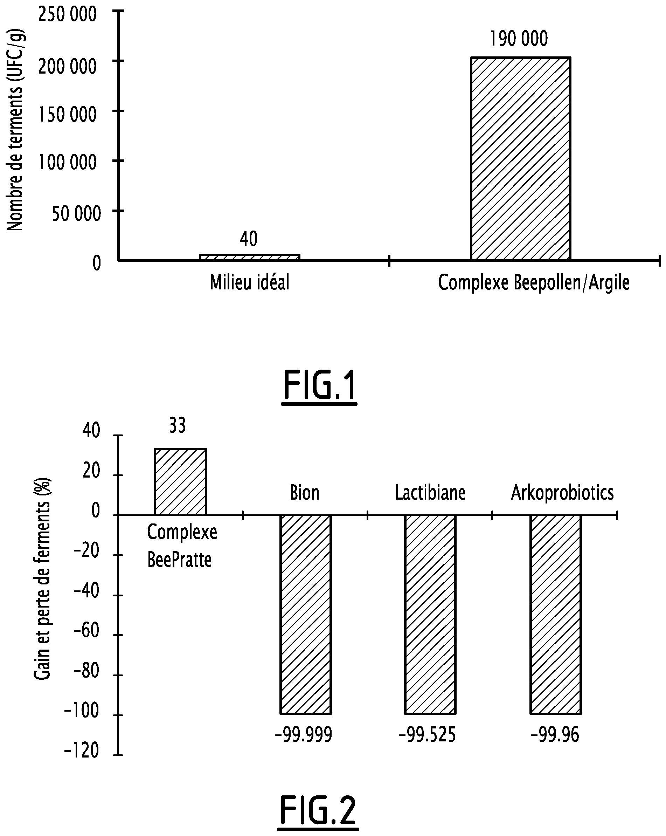

Les résultats illustrés à la Figure 1 démontrent que le complexe beepollen/argile permet la réactivation et la prolifération des bactéries.

### Exemple 2 : Mise en évidence de la résistance à l'acidité du complexe beepollen/argile par rapport à des produits commerciaux

Les trois produits commerciaux suivants ont été utilisés.

### Bion transit

Bion transit apporte une souche probiotique spécifique : *Lactobacillus plantarum 299v.*

Bion transit est destiné aux personnes dont l'intestin est sensible et qui sont sujettes à des troubles intestinaux fréquents : inconfort, gêne, pesanteur, ballonnements, transit irrégulier, etc.

Il est revendiqué sur le produit un apport de 10 000 000 000 (10 milliards) de probiotiques par gélule.

### Lactibiane référence 2,5G

Les souches probiotiques de Lactibiane Référence sont certifiées GRAS (Generally Regarded As safe) et déposées à la Collection Nationale de Cultures de Microorganismes de l'institut Pasteur. Il s'agit des quatre souches suivantes :
- *Bifidobacterium longum LA 101*
- *Lactobacillus acidophilus LA 102*
- *Lactococcus Lactis LA 103*
- *Streptococcus thermophilus LA 104*

Lactibiane référence 2,5G contribue au maintien de la flore intestinale, améliore le confort digestif et régule le transit dès la première semaine. Il peut aider en cas de troubles digestifs (ballonnements, etc.) et en prévention hivernale.

Il est revendiqué sur le produit un apport de 10 000 000 000 (10 milliards) de probiotiques par gélule.

### Arkoprobiotics défenses +

Arkoprobiotics defenses + contient une sélection de probiotiques lyophilisés provenant de plusieurs souches :
- *Lactobacillus paracasei*
- *Lactobacillus plantarum*
- *Lactobacillus rhamnosus*
- *Lactobacilus acidophilus*

Arkoprobiotics défense+ aide à renforcer les défenses naturelles de l'organisme, notamment pendant l'hiver et les changements de saison, ainsi qu'à rééquilibrer la flore intestinale. Il contribue à l'équilibre de la vitalité de l'organisme.

Il est revendiqué sur le produit un apport de 50 000 000 000 (50 milliards) de probiotiques par gélule.

### Mode opératoire :

Des gélules gastro-résistantes contenant le complexe beepollen/argile ou des souches bactériennes de produits commerciaux ont été fabriquées, au moyen du kit de préparation des gélules gastro-résistantes ApproPharm (254 chemin de la Farlède-83500 La Seyne sur Mer).

Le mode opératoire pour l'enrobage est le suivant :
- Tremper les gélules pleines de complexe beepollen/argile ou de produits commerciaux pendant 10 secondes dans le liquide.
- Laisser sécher sur un tamis, en remuant fréquemment pour éviter tout collage.
- Attendre 10 minutes et puis ajouter une 2^{e} couche : Tremper les gélules 5 secondes dans la solution, puis laisser sécher comme précédemment.
- Attendre 10 minutes et puis ajouter une 3^{e} couche : Tremper les gélules 5 secondes dans la solution, puis laisser sécher comme précédemment.
- Attendre 10 minutes et puis ajouter une 4^{e} couche : Tremper les gélules 5 secondes dans la solution, puis laisser sécher comme précédemment.
- Attendre 10 minutes et puis ajouter une 5^{e} couche : Tremper les gélules 5 secondes dans la solution, puis laisser sécher comme précédemment.

Des essais ont été conduits pour s'assurer de la résistance des gélules dans un milieu acide. Ils ont montré une excellente résistance au milieu acide puisque les enveloppes n'étaient pas rompues après un temps de 4 heures.

### Conditions expérimentales

Les gélules gastro-résistantes ainsi préparées ont été soumises aux conditions expérimentales suivantes.

Ainsi, les gélules
- ont été placées dans 500 mL (volume de l'estomac en préprandial)
- d'une solution acide de pH 2 (acidité du contenu stomacal)
- maintenues dans une étuve à la température de 37°C (température corporelle)
- pendant une durée totale de 4h (durée de transit au niveau stomacal)
- sous agitation mécanique (contraction de l'estomac).

A la fin de l'expérimentation, les gélules sont restées intègres (pas de rupture de la gélule entraînant une dispersion de son contenu), mais sont devenues légèrement perméables. Le contenu des gélules a été isolé et analysé pour réaliser le dénombrement des ferments lactiques.

### Dénombrement des ferments lactiques

Le dénombrement des ferments lactiques par QSA - Qualité Sécurité Alimentation - Site d'Agropole - BP 125 - 47931 Agen Cedex 9.

Le dénombrement des ferments lactiques présents dans le complexe beepollen/argile au sein des gélules gastro-résistantes et avant digestion indique 60 000 UFC par gramme. Après digestion, le complexe beepollen/argile présente une charge en bactéries lactiques de 80 000 UFC par gramme.

Pour les produits commerciaux avec mise en gélule gastro-résistante sans complexe, le dénombrement des ferments lactiques après digestion est le suivant :

| | |
|---|---|
| Bion Transit | 40 UFC/g |
| Lactibiane référence 2,5G | 190 000 UFC/g |
| Arkoprobiotics défenses + | 40 000 UFC/g |

Les variations lors de la digestion des ferments dans le complexe beepollen/argile et les trois produits commerciaux sont illustrées à la Figure 2. Les produits commerciaux formulés en gélules gastro-résistantes montrent que moins de 1% des ferments sont réactivés. Le support beepollen/argile sous forme de gélules gastro-résistantes permet d'augmenter la charge en ferments de 33% lors de la digestion.

### Exemple 3: Impact du complexe beepollen/argile sur la réactivation des bactéries lyophilisées

1) préparation des lots
   - des bactéries lyophilisées seules (souches commerciales pures)
      *versus*
   - les mêmes bactéries lyophilisées incluses dans le complexe beepollen/argile.

   Une quantité similaire de microorganismes a été incluse dans chaque lot, indépendamment de la quantité et/ou de la forme galénique du produit de référence (entre 1 000 000 et 2 000 000).
   Les bactéries lyophilisées incluses dans le complexe beepollen/argile ont été préparées de la façon suivante :
   Le volume de bactéries lyophilisées correspondant à une quantité de 1 000 000-2 000 000 a été ajouté au complexe. Le mélange a été homogénéisé à l'aide d'un agitateur pendant 30 secondes.
2) Les gélules gastro-résistantes ont été préparées selon la procédure de l'exemple 2.
3) Les gélules ont étés soumises aux conditions expérimentales suivantes. Ainsi, les gélules :
   - ont été placées dans 500 mL (volume de l'estomac en préprandial)
   - d'une solution acide de pH2 (acidité du contenu stomacal)
   - maintenues dans une étuve à la température de 37°C (température corporelle)
   - pendant une durée totale de 4h (durée de transit au niveau stomacal)
   - sous agitation mécanique (contraction de l'estomac).

Le dénombrement des ferments lactiques a été réalisé avant et après expérimentation par un laboratoire d'analyse microbiologique indépendant accrédité par le COFRAC (Comité Français d'Accréditation) pour le programme 59 - Analyses microbiologiques des produits agro-alimentaires - dans le cadre de la convention d'accréditation n°1344.

Les résultats obtenus sont les suivants :

### 1. Echantillons sans le complexe Beeopollen/argile

### Bactéries commerciales lyophilisées, avant mise en gélule, avant passage dans l'estomac (étapes 2 et 3 du protocole non réalisées) :

### Bactéries lyophilisées avant passage dans l'estomac :

| | |
|---|---|
| *Bifidobacterium longum* | 800 UFC/g |
| *Lactobacillus acidophilus* | 41 000 000 UFC/g |
| *Bifidobacterium bifidum* | 75 000 000 UFC/g |

### Bactéries commerciales lyophilisées, mises en gélules gastro-résistantes après passage dans l'estomac (étapes 1, 2 et 3 du protocole) :

| | |
|---|---|
| *Bifidobacterium longum* | 40 UFC/g |
| *Lactobacillus acidophilus* | 160 000 UFC/g |
| *Bifidobacterium bifidum* | 32 000 UFC/g |

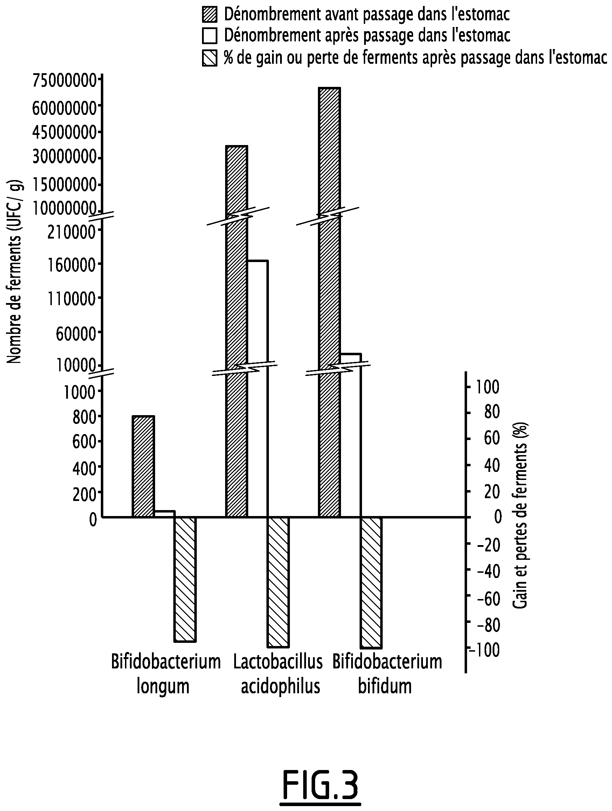

Les pourcentages de gain ou de perte des ferments lactiques après passage dans l'estomac ont été calculés. Ils sont représentés pour chaque bactérie, à la Figure 3.

### 2. Echantillons associés au complexe beepollen/argile

### Bactéries lyophilisées associées au complexe avant mise en gélule, avant passage dans l'estomac (étapes 2 et 3 du protocole non réalisées):

### Bactéries lyophilisées associées au complexe avant passage dans l'estomac :

| | |
|---|---|
| *Bifidobacterium longum* | 1 900 000 UFC/g |
| *Lactobacillus acidophilus* | 1 800 000 UFC/g |
| *Bifidobacterium bifidum* | 1 100 000 UFC/g |

### Bactéries lyophilisées associées au complexe, mises en gélules gastro-résistantes après passage dans l'estomac :

| | |
|---|---|
| *Bifidobacterium longum* | 7 400 000 UFC/g |
| *Lactobacillus acidophilus* | 2 600 000 UFC/g |
| *Bifidobacterium bifidum* | 2 600 000 UFC/g |

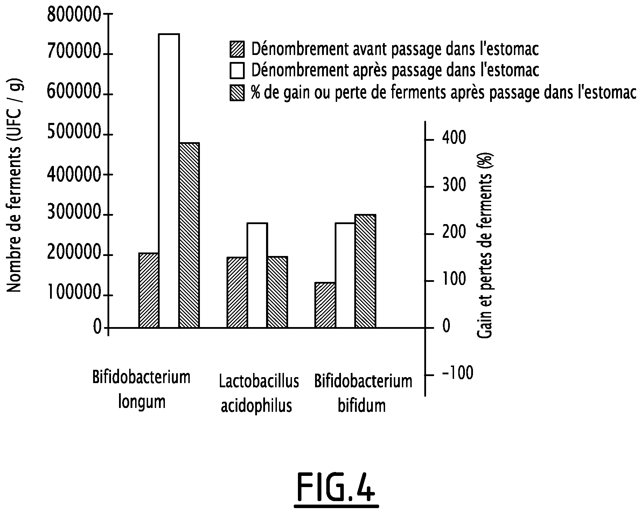

Les pourcentages de gain ou de perte des ferments lactiques après passage dans l'estomac ont été calculés. Ils sont représentés pour chaque bactérie, seule ou associée au complexe beepollen/argile, à la Figure 4.

Le complexe beepollen/argile formulé en gélules gastro-résistantes permet une augmentation des ferments des probiotiques lyophilisés lors de la digestion.

Les probiotiques lyophilisés formulés sous forme de gélules gastro-résistantes perdent au moins 95% de leurs ferments lors de la digestion.

Ces résultats montrent que :
✔ les effets de prolifération des ferments induits par le complexe beepollen/argile sur les bactéries incluses naturellement dans le beepollen frais peuvent être étendus aux bactéries lyophilisées.
✔ le complexe beepollen/argile est un support de choix pour la réactivation de probiotiques lyophilisés
✔ le complexe beepollen/argile sert de support de prolifération, d'implantation et donc d'administration de probiotiques.

### Exemple 4 : Etude intestinale

La démonstration que les bactéries qui arrivent au niveau intestinal ne sont pas dégradées par les différents sucs sécrétés et qu'elles adhèrent à la muqueuse intestinale est un enjeu de taille et constitue un potentiel scientifique et industriel.

En effet, lorsque les probiotiques arrivent au niveau de l'intestin, pour exercer leurs actions sur la santé, ils doivent résister à l'élévation du pH entre l'estomac et le duodénum, résister à l'impact des sels biliaires et des enzymes pancréatiques, enfin se fixer à la paroi intestinale pour pouvoir interagir avec le support en fonction des spécificités des bactéries.

La présente étude a pour but d'évaluer :
- la résistance des probiotiques aux variations de pH (gastrique et intestinal) et aux différents fluides (sels biliaires et enzymes pancréatiques)
- l'adhérence des probiotiques à la paroi intestinale.

Elle comporte donc deux expérimentations :
- une étude sur milieu artificiel permettant de mesurer la résistance des probiotiques aux variations physiologiques de pH et aux atteintes enzymatiques, de l'estomac au duodénum : ceci vient en quelque sorte compléter par le niveau intestinal les études précédentes qui étudiaient la résistance des probiotiques au milieu gastrique (et dont les résultats ont été rappelés précédemment) ;
- une étude ex-vivo sur explants d'intestin pour mesurer à la fois la survie des probiotiques dans la lumière intestinale et leur adhésion à la muqueuse intestinale.

### PROTOCOLE

### 1. Echantillons à tester

Les échantillons qui devront être testés lors de cette étude sont :
- Argile seule
- Pollen seul
- Probiotique seul (*Bifidobacterium longum*)
- Complexe beepollen/argile
- Complexe beepollen/argile + probiotiques
- Produits Bion, Lactibiane, Arkoprobiotics

Un témoin (sérum physiologique) sera réalisé en parallèle, en même temps que ces échantillons.

Pour des raisons d'homogénéité, tous les échantillons ont été mis en gélules de taille 1, conservées à 4°C.

Les exemples 1-3 ayant montré que la mise en gélules gastro-résistantes des probiotiques commerciaux comme de *Bifidobacterium longum* ne présentait pas d'intérêt quant à leur survie par rapport à l'utilisation de gélules classiques, seuls les échantillons comportant le complexe beepollen/argile et le complexe beepollen/argile associés aux probiotiques ont été conditionnés en gélules gastro-résistantes selon le protocole ci-après.
- Tremper les gélules pleines de complexe Beepollen/argile avec ou sans probiotiques pendant 10 secondes dans le liquide.
- Laisser sécher sur un tamis, en remuant fréquemment pour éviter tout collage.
- Attendre 10 minutes et puis ajouter une 2ème couche : Tremper les gélules 5 secondes dans la solution, puis laisser sécher comme précédemment.
- Attendre 10 minutes et puis ajouter une 3ème couche : Tremper les gélules 5 secondes dans la solution, puis laisser sécher comme précédemment.
- Attendre 10 minutes et puis ajouter une 4ème couche : Tremper les gélules 5 secondes dans la solution, puis laisser sécher comme précédemment.
- Attendre 10 minutes et puis ajouter une 5ème couche : Tremper les gélules 5 secondes dans la solution, puis laisser sécher comme précédemment.

### 2. Simulation in vitro de la phase stomacale

L'ensemble des échantillons est soumis au protocole de l'exemple 2.

Au niveau de l'estomac, les échantillons sont soumis à de nombreux paramètres physiologiques qui ont étés reproduits de la manière suivante:
- ils ont été dilués dans 500mL (volume de l'estomac en pré-prandial)
- d'une solution acide de pH2 (acidité du contenu stomacal)
- maintenus dans une étuve à la température de 37°C (température corporelle)
- pendant une durée totale de 4h (durée de transit au niveau stomacal)
- sous agitation (contraction de l'estomac).

Après incubation :
- dans le cas du complexe beepollen/argile associé aux *Bifidobacterium longum,* le contenu de ces gélules a été analysé d'un point de vue microbiologique ;
- dans le cas des gélules non gastro-résistantes, le liquide stomacal a également été analysé d'un point de vue microbiologique ;
- 62,5 mL de l'extrait de sucs biliaire et de suc pancréatique ont été ajoutés au liquide stomacal. Un échantillon de ce liquide (chyme intestinal) a été analysé d'un point de vue microbiologique.

### 3. Passage du pH gastrique au pH intestinal

A la fin des 4 heures d'incubation, 62,5 ml d'une solution constituée d'une solution de bicarbonate de sodium à 0.2M et d'extrait de bile à 1,2% et de suc pancréatique à 0.2% sont ajoutés au liquide gastrique.

### 4. Etude sur explants : étude préparatoire

### 4.1. Choix du modèle animal.

Le porc a été choisi comme modèle animal, cet animal ayant la physiologie digestive la plus proche de celle de l'Homme : son tractus gastro-intestinal lui est similaire du point de vue structural (Heinz et al., 1987, J. Gen. Virol. 68, 2495-2499).

### 4.2. Récupération de l'intestin de porc

Les intestins récupérés après abattage des animaux à destination alimentaire (dénués de toute pathologie) sont placés dans une solution physiologique. La solution est composée d'eau distillée et de chlorure de sodium (NaCl) dilué à 9 pour 1000 (= solution à 0,9 % de poids/volume de NaCl, soit 9 g/l). Elle contient 154 mEq/l de Na+ et de Cl- avant utilisation, puis l'intestin est lavé dans une solution de Ringer.

Des segments longitudinaux de 6 cm par 27 cm sont préparés dans le duodéno-jéjunum et conservés dans la solution de Ringer.

### 5. Simulation de la phase intestinale

Pour visualiser l'adhésion des microbactéries à la muqueuse intestinale, un appareil permettant de simuler la digestion sur des explants d'intestins de porc a été développé avec les paramètres suivants:
Le contenu du chyme gastrique ajusté au bon pH est placé dans un des réceptacles en acier inoxydable de 700mL. Le contenu est brassé en continu de manière à simuler les mouvements gastriques et intestinaux à l'aide d'une hélice (20 tours par minute).

Les intestins de porcs sont placés sur les plaques présentant les caractéristiques suivantes:
- des plaques inclinées (11°) en acier inoxydable de 6.8 cm sur 22 cm qui sont recouvertes d'une membrane souple et crantée permettant de modéliser le péristaltisme. Ce mouvement sera réalisé à l'aide d'un tapis roulant à la vitesse de 0,5cm/minute.

L'intestin est alors positionné sur la plaque. Lorsque l'intestin est correctement positionné sur la plaque, le tapis est activé et l'écoulement du liquide intestinal est lancé pour une durée de deux heures, dans les conditions suivantes :
- Maintien de l'intestin de porc à une température constante de 37°C.
- L'hygrométrie est contrôlée (80%) de manière à ne pas assécher l'intestin de porc.
- L'air interne de la chambre est constitué de 95% d'azote (N2) et 5% d'air.
- Maintien de l'intestin de porc à l'obscurité durant la phase expérimentale.

### 6. Mesures

Au bout des 2 heures, les intestins sont
- enlevés de leurs supports ;
- découpés en sections transversales ;
- observées au microscope et raclées pour réaliser un dénombrement microbiologique :
   Le liquide passé sur l'intestin et recueilli dans les bacs de récupération est analysé d'un point de vue microbiologique.

Le liquide avant passage sur l'intestin est également analysé d'un point de vue microbiologique. Sur les échantillons, on réalise une analyse microbiologique de la flore lactique (NF ISO 15214).

Les résultats relatifs aux comptages microbiologiques avant, pendant et après passage sur l'intestin sont transmis en Unité Formant des Colonies par gramme (UFC - Units Forming Colony per gram). Cela permet de comparer les différents niveaux et d'évaluer les éventuelles proliférations. Les ratios: surfaces d'intestins analysés par rapport à la surface totale de l'intestin sont pris en considération.

Les morphologies cellulaires ont été évaluées. L'intégrité du grain de pollen est également évaluée.

### RESULTATS ET DISCUSSIONS

L'objectif de cette étude est d'évaluer la résistance des probiotiques à l'acidité gastrique, puis à l'élévation du pH au cours du transit, à la mise en contact avec les sels biliaires et les enzymes pancréatiques, leur survie aux différents fluides intestinaux et leur adhérence à la paroi intestinale.

### 1. Evaluation de la survie de ferments à la phase stomacale.

Les exemples 1-3 montrent que la digestion ne permet pas aux bactéries de survivre s'ils ne sont pas associés au complexe beepollen/argile.

Dans ce même contexte physiologique, le complexe beepollen/argile favorise une augmentation de la quantité de probiotiques ingérés. La biocharge augmente en présence du complexe, alors que, dans le même temps, les probiotiques (*Bifidobacterium longum*) sans la protection du complexe sont quasiment tous tués.

Ainsi, le passage au niveau de l'estomac s'inscrit comme un facteur favorable à la prolifération des bactéries si elles sont protégées par le complexe. Au contact de l'eau contenue dans l'estomac, la gélule gastro-résistante devient perméable, l'eau pénètre et apporte l'humidité dont les bactéries ont besoin pour être réactivées et entamer la phase de prolifération, sous réserve que les bactéries aient, comme c'est le cas avec le complexe, la réserve nutritive suffisante.

Cette étape cruciale de réactivation et prolifération au sein du complexe constitue un axe central et prépondérant dans l'activité probiotique du complexe.

### 2. Evaluation de la survie de ferments aux sels biliaires et aux sucs pancréatiques

L'acidité gastrique et les secrétions bilio-pancréatiques constituent les principaux mécanismes endogènes d'inactivation des bactéries probiotiques ingérées.

Le complexe favorise le développement intra-corpus de nouvelles bactéries dès la phase stomacale, amélioré encore lors de la mise en contact avec les sucs biliaires et enzymatiques. Les dénombrements de la flore contenue dans le liquide intestinal avant passage sur l'intestin permet de déterminer l'apport du complexe dans la survie des probiotiques au contact de la bile et de sucs pancréatiques. Mieux que survivre, les probiotiques continuent et accentuent leur prolifération.

**Les résultats obtenus montrent que sans complexe, il persiste 78 750 probiotiques *Bifidobacterium longum* au niveau du liquide intestinal. Avec le complexe contenant ces bactéries, on en dénombre 101 250 000. Ceci représente un facteur de respectivement 46, >100 000 000, 600 par rapport aux produits commerciaux testés sans le complexe (respectivement Bion, Lactibiane, Arkoprobiotics).**

Au-delà de les maintenir en vie, le complexe beepollen/argile permet aux bactéries lactiques de se développer dans le liquide intestinal.

Mieux encore : alors que la survie et la prolifération en milieu stomacal constitue une caractéristique essentielle et déterminante, le phénomène de résistance et de prolifération intra-corpus s'accentue lors du contact avec la bile. **On constate ainsi une augmentation d'un facteur 7,79 entre la fin de la phase stomacale et le début de la phase intestinale.**

Ces résultats indiquent que non seulement, les bactéries sont protégées par le complexe, mais ce dernier assure en outre leur développement.

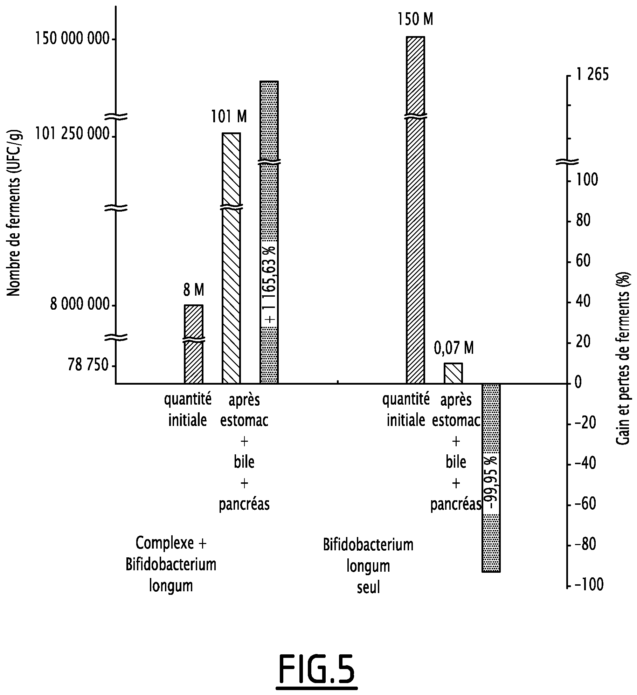

Comme illustré à la Figure 5, il ne reste en effet que 0,05% des probiotiques initiaux après le passage gastrique et l'impact de la bile et des enzymes pancréatiques s'ils ne sont pas protégés. Au contraire, **soumis aux mêmes conditions, les mêmes probiotiques présentent un gain de 1 165,63 % s'ils sont inclus dans le complexe.**

Ces résultats montrent que le complexe beepollen/argile protège les bactéries lors de la digestion que ce soit vis-à-vis de l'acidité gastrique ou des sucs pancréatiques et biliaires et favorise leur développement.

### 3. Evaluation des probiotiques présents dans le liquide intestinal recueilli en fin d'écoulement

Il est également important d'observer la charge contenue dans le liquide intestinal qui va poursuivre son trajet le long de l'intestin et du colon.

**Les résultats obtenus montrent que la biocharge contenue dans le liquide intestinal recueilli à la fin de l'écoulement est de 77 millions de ferments avec le complexe. Sans le complexe, il ne reste que 3,4 millions de bactéries dans le liquide intestinal, soit 22,65 fois moins.**

**Le complexe permet d'avoir respectivement 23, 424 et 636 fois plus de bactéries que les produits commerciaux (Bion, Lactibiane et Arkoprobiotics respectivement).**

### 4. Evaluation de l'adhésion du complexe sur la muqueuse intestinale.

Un autre point déterminant de l'étude concerne l'adhésion du complexe et des bactéries sur la muqueuse intestinale. Pour avoir une action bénéfique sur la muqueuse, les probiotiques doivent y adhérer.

### 4.1. Evaluation macroscopique

Des clichés ont été pris avant et après la phase intestinale de l'étude, c'est-à-dire avant et après l'écoulement du liquide intestinal qui contient le complexe avec les *Bifidobactérium longum.* On remarque une différence très significative entre la muqueuse avant et après écoulement. En effet, un tapis orangé est visible sur toute la surface de l'intestin ayant été en contact avec le liquide intestinal. Les photos montrent sans ambiguïté une présence jaune caractéristique de pollen, cette fois libéré de son "manteau" d'argile.

Ce constat visuel semble confirmer:
- la présence du complexe sur la paroi intestinale malgré l'écoulement et l'effort mécanique associé,
- la capacité du complexe à se séparer *in situ.*

L'observation au microscope de l'intestin permet de visualiser avec précision la présence isolée d'argile et de pollen (entouré ou non de son « manteau » d'argile).

Un plus fort grossissement permet de visualiser dans le mucus des grains de pollen avec leur "manteau" d'argile, mais aussi et surtout des grains de pollen débarrassés de cette structure argileuse, faisant la démonstration de la capacité des grains de pollen à se libérer de leur gangue argileuse. Les grains non encore libérés à ce stade vont pouvoir le faire tout le long du parcours intestinal.

Ces observations indiquent que les attaches entre le pollen et l'argile peuvent se rompre au niveau intestinal et libérer les ferments et le contenu du pollen. Comme certains grains de pollen sont encore recouverts d'argile, ils peuvent continuer leur trajet dans l'intestin et se dissocier progressivement le long du tube digestif.

### 4.2. Evaluation microbiologique

En raison de l'hétérogénéité d'un intestin de porc, les données microbiologiques sont analysées avec la moyenne des dénombrements des 3 segments rapportés à la surface de l'intestin étudié.

**A la fin de l'écoulement du liquide intestinal sur l'intestin (fin de la digestion), la biocharge contenue dans le mucus intestinal prélevé par raclage de la muqueuse est de 582 650 UFC/g avec les bactéries lyophilisées (*Bifidobacterium longum*) contre 5 160 000 dès lors que ces dernières sont incluses dans le complexe. On retrouve donc 9 fois plus de bactéries au niveau de la muqueuse intestinale lorsque le complexe beepollen/argile est utilisé comme vecteur. Le complexe permet d'avoir respectivement 69, 301 et 818 fois plus de bactéries au niveau de la muqueuse intestinale que les produits commerciaux (Lactibiane, Bion et Arkoprobiotics respectivement).**

Dans le liquide récolté après passage sur l'intestin, le rapport entre les bactéries présentes dans le complexe et les bactéries sans complexe était de plus de 22 fois. Cette différence entre ces deux rapports s'explique par le fait que la très importante biocharge du liquide intestinal lors de l'utilisation du complexe comme support des probiotiques a saturé en microferments la muqueuse intestinale.

En outre, ceci permet aux bactéries contenues dans le liquide intestinal (et qui vont continuer à se multiplier par la suite du trajet intestinal) de coloniser les parties suivantes du tube digestif.

Le complexe beepollen/argile constitue donc un bon facteur favorisant l'adhésion des probiotiques à la muqueuse intestinale.

### 4.3. Evaluation des empreintes

La flore lactique présente une meilleure adhérence lorsqu'elle est associée au complexe. On observe également une action périphérique sur la flore totale. Outre l'intérêt du complexe pour la flore lactique, il semble agir sur la maîtrise de la flore totale, cette dernière étant significativement moins importante en présence du complexe.

Le développement de la flore totale est totalement différent sur les empreintes des segments d'intestins avec ou sans complexe. Les *Bifidobacterium longum* seuls génèrent des empreintes avec un développement de la flore totale très important (indénombrable sur l'empreinte). En revanche, avec le complexe associé avec les *Bifidobacterium longum,* très peu de colonies (entre 10 et 20) se développent. A l'opposé, on observe un important développement de la flore lactique. Ceci signifie que la flore que l'on souhaite implanter (les probiotiques) vient bien en compétition avec la flore totale au niveau de la muqueuse digestive où elle s'implante au détriment de cette dernière : c'est précisément ce qui est souhaité lors d'utilisation de probiotiques.

Les conclusions globales des résultats mentionnés aux paragraphes 2 à 4 de cette partie sont donc les suivantes :
- le complexe permet une survie des probiotiques aux attaques des sucs biliaires et pancréatiques ;
- mieux, il permet une très importante multiplication de ces probiotiques, ceux-ci étant adaptés à ce milieu ;
- le complexe permet une adhésion de ces probiotiques à la muqueuse intestinale ;
- le pollen peut progressivement se détacher du complexe pour libérer les bactéries ;
- la très grande quantité de la flore apportée et sa grande viabilité lui permettent de s'implanter dans la flore locale.

### 5. Evaluation de la synergie des ingrédients du complexe beepollen/argile

Parmi les échantillons utilisés dans cette étude, l'un comportait l'argile seule et un autre le pollen seul. Il est donc possible de juger de l'intérêt du complexe en comparant l'effet attendu en calculant avec une pondération l'effet des constituants pris isolément et les résultats obtenus par l'étude : on constate l'effet synergique du complexe.

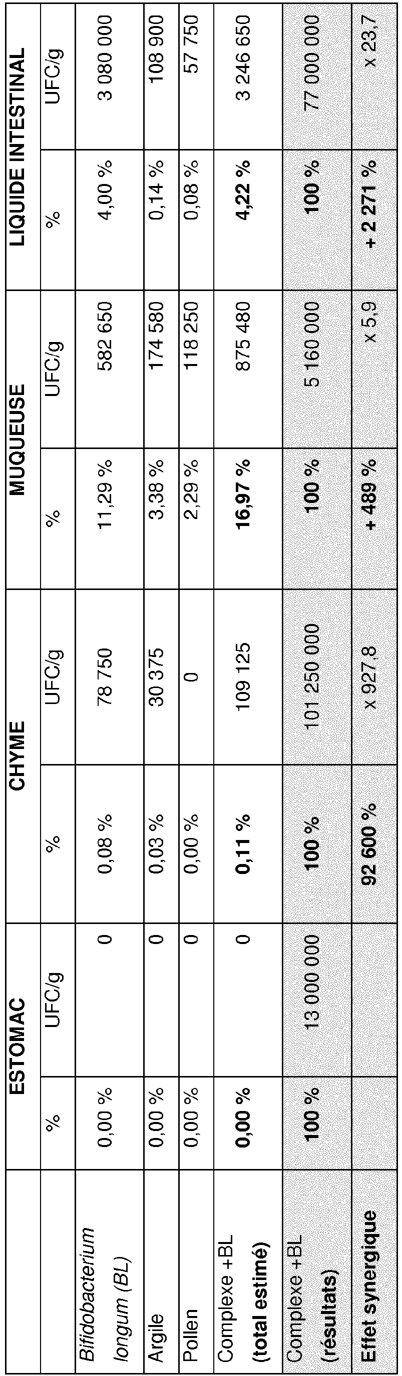

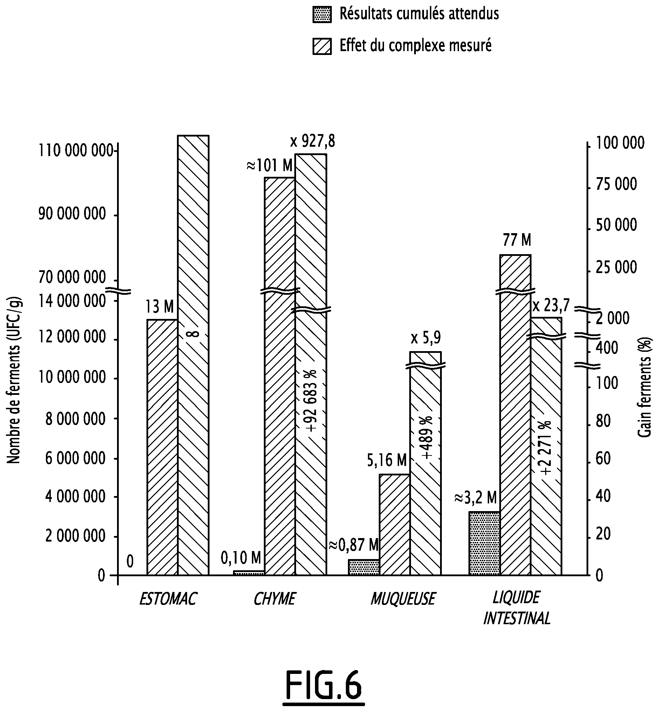

On peut donc visualiser ainsi l'effet synergique à tous les stades du complexe :
L'effet synergique du beepollen et de l'argile sur les gains de ferments dans l'estomac, chyme, muqueuse et liquide intestinal est illustré à la Figure 6.

Le complexe permet d'augmenter le nombre de bactéries lactiques de 92 683 % dans le chyme, de 489 % au niveau de la muqueuse intestinale et de 2271 % dans le liquide intestinal, par rapport aux valeurs estimées.

Ces résultats *ex-vivo* obtenus sur des intestins de porc ont permis d'évaluer la résistance des probiotiques à l'acidité gastrique, puis à l'élévation du pH au cours du transit, à la mise en contact des sels biliaires et des sucs pancréatiques et leur survie aux différents fluides intestinaux ainsi que leur adhérence à la paroi intestinale.

L'apport bénéfique du complexe beepollen/argile a été démontré sur toutes les phases de la digestion.

### CONCLUSIONS:

Lors de la phase stomacale, lorsqu'il est conditionné en gélules gastro-résistantes, le complexe beepollen/argile permet aux ferments qui y sont associés d'être réactivés. C'est un support de choix à la réactivation et à la prolifération de "nouvelles" bactéries lactiques.

Lors de la phase intestinale, le complexe est dans un premier temps mis en contact avec la bile et les sucs pancréatiques sans que cela n'affecte la viabilité cellulaire puisqu'au contraire, la capacité de prolifération demeure et croît.

Lors du transit intestinal, le complexe adhère facilement à la muqueuse. Les observations microscopiques montrent que les grains de pollen sont libérés de leur manteau d'argile, libérant *in situ* leur cytoplasme. Le dénombrement des ferments sur la muqueuse intestinale permet d'affirmer que le complexe libère bien sa biocharge au niveau intestinal tant sur la paroi intestinale que dans le flux de la lumière intestinale. L'intérêt majeur et différenciant du complexe consiste en l'action synergique de certaines caractéristiques intrinsèques des composants comme l'effet adsorbant de l'argile. L'apport nutritif du pollen est ainsi complété par sa capacité à s'opposer à l'adsorption irréversible des bactéries au niveau de l'argile. Cette augmentation de probiotiques sur la surface de la muqueuse intestinale joue également un rôle sur la flore totale dont elle réduit le développement.

## Revendications

1. Composition comprenant :
- un ou plusieurs microorganismes à visée probiotique caractérisés et mis sous culture avant leur formulation et qui, lorsqu'ils sont délivrés vivants en quantité suffisante à l'intestin, confèreraient un bénéfice pour la santé et/ou la nutrition de l'hôte,
ajoutés à
- un complexe à base de beepollen et d'argile.

2. Composition selon la revendication 1 telle que lesdits microorganismes sont lyophilisés.

3. Composition selon la revendication 1 ou 2 telle que lesdits microorganismes sont choisis parmi les bactéries lactiques.

4. Composition selon la revendication 1, 2 ou 3 telle que lesdits microorganismes sont choisis parmi les bactéries des genres *Bifidobacterium spp* et *Lactobacillus spp.*

5. Composition selon l'une quelconque des revendications précédentes comprenant :
- de 0,1% à 70% de microorganismes lyophilisés ; et
- de 30% à 99,9% dudit complexe,
les pourcentages s'entendant en poids.

6. Composition selon l'une quelconque des revendications précédentes comprenant :
- de 3% à 30% de microorganismes lyophilisés ; et
- de 70% à 97% dudit complexe
les pourcentages s'entendant en poids.

7. Composition selon l'une quelconque des revendication précédentes telle que le dit complexe comprend :
- de 20 % à 40 % de beepollen et
- de 60 % à 80 % d'argile.

8. Composition selon l'une quelconque des revendications précédentes telle qu'elle se présente sous forme d'une poudre.

9. Composition selon l'une quelconque des revendications précédentes en formulation gastrorésistante.

10. Composition selon la revendication 9 formulée en gélules gastrorésistantes.

11. Procédé de préparation d'une composition selon l'une quelconque des revendications 1 à 10 comprenant :
- le mélange de beepollen et d'argile ; et
- l'ajout et le mélange de microorganismes.

12. Composition alimentaire, cosmétique, et/ou galénique comprenant une composition selon l'une quelconque des revendications 1 à 10.

13. Composition alimentaire selon la revendication 12 telle qu'il s'agit d'un aliment, un aliment fonctionnel, ou un complément alimentaire.

14. Composition pharmaceutique comprenant une composition selon l'une quelconque des revendications 1 à 10.

15. Utilisation d'un complexe à base de beepollen et d'argile pour améliorer la biodisponibilité et/ou l'activité de microorganismes à visée probiotique caractérisés et mis sous culture avant leur formulation et qui, lorsqu'ils sont délivrés vivants en quantité suffisante à l'intestin, confèreraient un bénéfice pour la santé et/ou la nutrition de l'hôte.

16. Composition pharmaceutique selon la revendication 14 pour son utilisation thérapeutique pour améliorer la biodisponibilité et/ou l'activité de microorganismes à visée probiotique caractérisés et mis sous culture avant leur formulation et qui, lorsqu'ils sont délivrés vivants en quantité suffisante à l'intestin, confèreraient un bénéfice pour la santé et/ou la nutrition de l'hôte.

## Patentansprüche

1. Zusammensetzung, enthaltend:
- einen oder mehrere Mikroorganismen mit probiotischer Zielrichtung, vor ihrer Formulierung charakterisiert und kultiviert, und die, wenn sie in ausreichender Menge lebend dem Darm zugeführt werden, einen Nutzen für die Gesundheit und/oder die Ernährung des Wirts mit sich bringen würden,
hinzugefügt zu
- einem Komplex auf der Basis von Bienenpollen und Ton.

2. Zusammensetzung nach Anspruch 1, derart, dass die Mikroorganismen lyophilisiert sind.

3. Zusammensetzung nach Anspruch 1 oder 2, derart, dass die Mikroorganismen ausgewählt sind aus den Milchsäurebakterien.

4. Zusammensetzung nach Anspruch 1, 2 oder 3, derart, dass die Mikroorganismen ausgewählt sind aus den Bakterien der Gattung *Bifidobacterium spp* und *Lactobacillus spp.*

5. Zusammensetzung nach einem beliebigen der vorhergehenden Ansprüche, enthaltend:
- von 0,1% bis 70% lyophilisierter Mikroorganismen; und
- von 30% bis 99,9% des Komplexes,
wobei die Prozente als Gewichtsprozente zu verstehen sind.

6. Zusammensetzung nach einem beliebigen der vorhergehenden Ansprüche, enthaltend:
- von 3% bis 30% lyophilisierter Mikroorganismen; und
- von 70% bis 97% des Komplexes,
wobei die Prozente als Gewichtsprozente zu verstehen sind.

7. Zusammensetzung nach einem beliebigen der vorhergehenden Ansprüche, derart, dass der Komplex enthält:
- von 20% bis 40% an Bienenpollen; und
- von 60% bis 80% Ton.

8. Zusammensetzung nach einem beliebigen der vorhergehenden Ansprüche, derart, dass sie eine Pulverform aufweist.

9. Zusammensetzung nach einem beliebigen der vorhergehenden Ansprüche als gastroresistente Formulierung.

10. Zusammensetzung nach Anspruch 9, formuliert als gastroresistente Kapseln.

11. Verfahren zur Herstellung einer Zusammensetzung nach einem beliebigen der Ansprüche 1 bis 10, enthaltend:
- die Mischung aus Bienenpollen und Ton; und
- den Zusatz und die Mischung an Mikroorganismen,

12. Nahrungsmittel-, Kosmetik- und/oder galenische Zusammensetzung, umfassend eine Zusammensetzung nach einem beliebigen der Ansprüche 1 bis 10.

13. Nahrungsmittelzusammensetzung nach Anspruch 12, derart, dass es sich um ein Nahrungsmittel, ein funktionelles Lebensmittel oder ein Nahrungsergänzungsmittel handelt.

14. Pharmazeutische Zusammensetzung, eine Zusammensetzung nach einem beliebigen der Ansprüche 1 bis 10 enthaltend.

15. Verwendung eines Komplexes auf Basis von Bienenpollen und Ton zur Verbesserung der Bioverfügbarkeit und/oder der Aktivität von Mikroorganismen mit probiotischer Zielrichtung, vor ihrer Formulierung charakterisiert und kultiviert, und die, wenn sie in ausreichender Menge lebend dem Darm zugeführt werden, einen Nutzen für die Gesundheit und/oder die Ernährung des Wirts mit sich bringen würden.

16. Pharmazeutische Zusammensetzung nach Anspruch 14 zur therapeutischen Verwendung in der Verbesserung der biologischen Verfügbarkeit und/oder der Aktivität von Mikroorganismen mit probiotischer Zielrichtung, vor ihrer Formulierung charakterisiert und unter Kultur gesetzt, und die, wenn sie in ausreichender Menge lebend dem Darm zugeführt werden, einen Nutzen für die Gesundheit und/oder die Ernährung des Wirts mit sich bringen würden.

## Claims

1. A composition comprising:
- one or several micro-organisms with a probiotic purpose characterized and cultivated before their formulation and that when they are delivered alive in a sufficient amount to the intestine, would give a health and/or nutrition benefit to the host added to
a complex based on beepollen and clay.

2. The composition according to claim 1, such that said micro-organisms are freeze-dried.

3. The composition according to claim 1 or 2, such that said micro-organisms are selected from lactic bacteria.

4. The composition according to claim 1, 2 or 3, such that said micro-organisms are selected from bacteria of the *Bifidobacterium spp* and *Lactobacillus spp* genera.

5. The compositions according to any of the proceeding claims, comprising,
- from 0.1 % to 70% of freeze dried micro-organisms; and
- from 30% to 99.9% of said complex,
the percentages being understood by weight

6. The composition according to any of the preceding claims, comprising:
- from 3% to 30% of freeze-dried micro-organisms; and
- from 70% to 97% of said complex,
the percentages being understood by weight.

7. The compositions according to any of the preceding claims, such that said complex comprises:
- from 20% to 40% of beepollen and
- from 60% to 80% of clay.

8. The composition according to any of the preceding claims such that it appears as a powder.

9. The composition according to any of the preceding claims in a gastro-resistant formulation.

10. The composition according to claim 9, formulated as gastro-resistant gelatin capsules.

11. A method for preparing a composition according to any of claims 1 to 10 comprising:
- mixing beepollen and clay; and
- adding and mixing micro-organisms

12. A food, cosmetic, and/or galenic composition comprising a composition according to any of claims 1 to 10.

13. The food composition according to claim 12, such that it is a foodstuff, a functional foodstuff or a food supplement.

14. A pharmaceutical composition comprising a composition according any of claims 1 to 10.

15. The use of a complex based on beepollen and clay for improving bioavailability and/or activity of micro-organisms with a probiotic purpose characterized and cultivated before their formulation and that when they are delivered alive in a sufficient amount to the intestine, would give a health and/or nutrition benefit to the host.

16. The pharmaceutical compositions according to claim 14 for its use in order to improve bioavailability and/or activity of micro-organisms with a probiotic purpose characterized and cultivated before their formulation and that when they are delivered alive in a sufficient amount to the intestine, would give a health and/or nutrition benefit to the host.
